# EUROPEAN PATENT APPLICATION

(11) **EP 3 287 128 A1**
(43) Date of publication of application: **28.02.2018**
(21) Application number: 16783123.9
(22) Date of filing: 18.04.2016
(51) Int. Cl.: A61K 31/085, A61P 9/00, A61P 11/00, A61P 11/06, A61P 43/00

(54) **CHRONIC RESPIRATORY DISEASE THERAPEUTIC AGENT AND CARDIAC FIBRILLATION SUPPRESSING COMPOSITION**

(30) Priority: 23.04.2015 JP 2015088697; 13.10.2015 WO PCT/JP2015/078962
(71) Applicant: NIPPON HYPOX LABORATORIES INCORPORATED, Tokyo 192-0354 (JP)
(72) Inventor: MIKI, Tokutaro, Hachioji-shi Tokyo 192-0354 (JP); NISHIKAWA, Hiroshi, Minamikoma-gun Yamanashi 409-2212 (JP); KANG, Jong-Koo, Cheongju-si Chungcheongbuk-do 28424 (KR); SUGIYAMA, Satoru, Nagoya-shi Aichi 464-0833 (JP)
(74) Representative: Green, Mark Charles
(86) International application number: PCT/JP2016/062256
(87) International publication number: WO 2016/171102

(57) **Abstract**

A novel agent which is effective in the prevention or treatment of a chronic respiratory disease such as COPD, interstitial pneumonia and asthma is provided. The therapeutic agent for a chronic respiratory disease comprises, as an active ingredient, a hydroquinone derivative represented by general formula (1) wherein R¹ represents an alkyl group having 4 to 8 carbon atoms, and R² represents a hydrogen atom, an alkylcarbonyl group having 2 to 6 carbon atoms or an alkoxycarbonyl group having 2 to 6 carbon atoms.

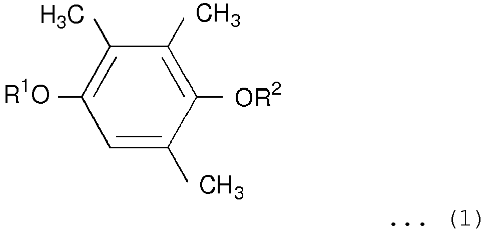

## Description

### Technical Field

The present invention relates to a therapeutic agent for a chronic respiratory disease, a food composition for prevention or improvement of a chronic respiratory disease, a composition for inhibiting cardiac fibrosis and a composition for alleviating a side effect of an agent, comprising a specific hydroquinone derivative as an active ingredient.

### Background Art

Chronic respiratory diseases are noninfective chronic diseases of the respiratory tract and lung tissue, and examples of the chronic respiratory diseases mainly include chronic obstructive pulmonary disease (COPD), asthma and interstitial pneumonia. Among these, the chronic obstructive pulmonary disease (COPD) is an inflammatory disease of the lung mainly caused by long term inhalation of toxic substances such as tobacco smoke or polluted air, and exhibits progressive airflow obstruction. Prevalence and the mortality rate of COPD is in high level worldwide (the 4th cause of death in the world, survey by WHO, 2004) and the number of patients is expected to increase over the next few decades. A considerable number of potential patients are thought to exist, because the disease, COPD, is generally not widely recognized.

One of chronic respiratory diseases, interstitial pneumonia is caused by fibrosing of inflammatory tissue as a result of inflammation of interstitial tissue of lungs. In lungs, as much as 300 million alveoli take air and gas exchange is performed through capillaries winding around these alveoli, and the tissue which surrounds and supports them is interstitium. When the interstitium fibroses, whole lungs become stiff and normal expansion and contraction of lungs are obstructed, and thus vital capacity is decreased and the efficiency of gas exchange between alveoli and capillaries is also decreased at the same time. The interstitial pneumonia includes the interstitial pneumonia whose causes of onset have been proved and the interstitial pneumonia whose causes have not been identified, and one cause of onset which has been proved is agents. For instance, bleomycin is an anticancer antibiotic separated from Streptomyces verticillus and is used as a therapeutic agent for many types of cancers, because myelosuppression action, which is frequently observed in the use of an anticancer agent, is less and nausea and vomiting are relatively mild in the use of bleomycin. However, bleomycin has severe side effects which tend to induce the interstitial pneumonia. Therefore, bleomycin is also used to produce disease-model animals of interstitial pneumonia. In addition to the bleomycin, numerous agents such as anticancer agents such as gefitinib, erlotinib, cetuximab, panitumumab and bortezomib etc., platinating agents (anticancer agents) such as cisplatin and oxaliplatin etc., immunosuppressive agents such as cyclophosphamide, azathioprine, tacrolimus and penicillamine etc., antirheumatic drugs such as methotrexate, salazosulfapyridine and leflunomide etc., vasodilators such as hydralazine etc., Kampo medicines such as shosaikoto etc., antiarrhythmic agents such as amiodarone etc. as well as interferon, antimicrobial agents, antiepileptic drugs and diuretics etc. are known to be a causative agent of interstitial pneumonia. Further, inhalation of powders of a mineral, pottery or stone etc. and asbestos etc., radiation exposure, collagen diseases and infectious diseases are known to be the causes of interstitial pneumonia. The idiopathic interstitial pneumonia whose causes cannot be identified is designated as a specific (intractable) disease, by the government.

Like the interstitial pneumonia described above, fibrosing diseases of organs include many intractable diseases, and identification of the causes is difficult or the method of treatment is not established in many of them. When the fibrosis of organ tissue proceeds, the whole organ becomes stiff and, in the case of a hollow organ, normal expansion and contraction become difficult, leading to dysfunction. Examples of the fibrosing diseases of a hollow organ include, in addition to the interstitial pneumonia described above, cardiomyopathy in the heart, and the interstitial pneumonia and the cardiomyopathy can be fatal diseases because both the lungs and the heart are important organs in which dysfunction directly leads to death.

The cardiomyopathy develops when myocardial cells necrotize and are replaced by a fibrotic tissue as a result of inflammation and/or degeneration in myocardial cells due to various causes. When the cardiac tissue fibroses, normal contraction functions are lost and the function of heart as a pump which sends blood to the whole body will be seriously disturbed. The cardiomyopathy also includes the cardiomyopathy whose causes have been proved and the cardiomyopathy whose causes have not been proved, and same as the interstitial pneumonia described above, it is known to be caused by the administration of agents. For instance, doxorubicin (adriamycin in another name), an anthracyclin anticancer agent, is an anticancer antibiotic extracted from Streptomyces Peucetius var. Caecius and is clinically used as a therapeutic agent for various types of cancers because it has a strong and broad anticancer spectrum. However, anthracyclin anticancer agents including doxorubicin have severe side effects which induce myocardial disorder in a dose-dependent manner. Specifically, it is known that myocardium gradually fibroses and whole myocardium becomes stiff and exhibits same manifestation with cardiomyopathy with the increase of the total dose of doxorubicin. Therefore, doxorubicin is also used to produce disease-model animals of cardiomyopathy. Further, it is known that viral infection, diabetes, obesity, thyroid diseases and alcohol etc. may also cause cardiomyopathy.

Meanwhile, the hydroquinone derivative represented by the following general formula (1) is a substance having strong anti-oxidant action and NO production inhibitory action. Patent Literatures 1 to 4 disclose an antioxidant (Patent Literature 1), a composition for treating arteriosclerosis (Patent Literature 2), a therapeutic agent for neurodegenerative diseases (Patent Literature 3) and an inhibitor of hepatic fibrosis (Patent Literature 4) comprising this hydroquinone derivative as an active ingredient.

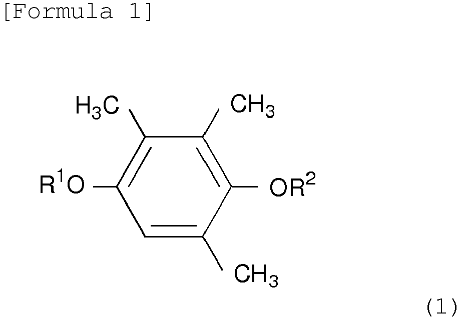

### Citation List

### Patent Literature

Patent Literature 1: Japanese Patent Laid-Open No. 5-301836
Patent Literature 2: Japanese Patent Laid-Open No. 2002-241366
Patent Literature 3: Japanese Patent Laid-Open No. 2009-102262
Patent Literature 4: Japanese Patent Laid-Open No. 2009-256226

### Summary of Invention

### Technical Problem

As described above, in COPD, inflammatory response of a respiratory tract or lungs is enhanced due to toxic substances, and there is no curative treatment method and only symptomatic treatment with a bronchodilator or an expectorant is conducted. Therefore, an agent which can inhibit the inflammatory response of a respiratory tract or lungs and stop the progression of the condition of COPD so that the condition dose not lead to a serious pathological condition has been sought. Since the fibrosing diseases such as interstitial pneumonia and cardiomyopathy, chronic respiratory diseases, are caused by various causes, the effective method of treatment is still under study. While only pirfenidone is approved as effective in Japan as a therapeutic agent of interstitial pneumonia, there was a problem that it has a side effect which increases the risk of photosensitivity or skin cancer. The main treatment of cardiomyopathy is surgical treatment such as cardiac transplantation and a ventricular assist device, and curative medical treatment has been sought.

Meanwhile, it is disclosed in each of the Patent Literature that the hydroquinone derivative disclosed in Patent Literatures 1 to 4 can be used as an antioxidant and can also be used as a therapeutic agent for arteriosclerosis, neurodegenerative disease and hepatic fibrosing disease, but discussion about the use in pulmonary or cardiac fibrosis diseases has not been done and its efficacy has been unknown.

Further, the pulmonary and cardiac fibrosis diseases induced by an agent develop as a side effect of an agent administered to a patient in expectation of the original efficacy of the agent. For instance, both bleomycin and the anthracyclin anticancer agent doxorubicin are used as a typical therapeutic agent in anticancer agent treatment because they have a broad anticancer spectrum, however, they induce pulmonary or cardiac fibrosis diseases, respectively, as a side effect. Therefore, administration of the agents may be stopped due to the occurrence of side effects, or the use of the agents is limited, for example, the total dose is limited to prevent side effects, despite their excellent original anticancer therapeutic effect. Thus, there was a problem that, despite the existence of effective therapeutic agents for severe diseases, the therapeutic agents cannot be used sufficiently due to side effects such as interstitial pneumonia and myocardial disorder.

The present invention was made in light of above mentioned points and an object of the invention is to provide a novel agent which is effective in the prevention or treatment of a chronic respiratory disease such as COPD, interstitial pneumonia and asthma.

Another object of the present invention is to provide a novel agent which is effective in the prevention or treatment of cardiac fibrosis diseases.

Another object of the present invention is to provide an novel agent which is effective in the prevention or treatment of pulmonary or cardiac fibrosis diseases developed as side effects of administration of a therapeutic agent or in reduction of the side effects.

### Solution to Problem

The present inventors have found that the hydroquinone derivative represented by the general formula (1) has action which inhibits COPD, asthma and interstitial pneumonia and excretes sputum as well as action which inhibits myocardial disorder as a result of intensive research in light of such a situation, thereby completing the present invention.

To solve the above mentioned problems, the therapeutic agent for a chronic respiratory disease according to the present invention comprises, as an active ingredient, the hydroquinone derivative represented by the following general formula (1) wherein R¹ represents an alkyl group having 4 to 8 carbon atoms, and R² represents a hydrogen atom, an alkylcarbonyl group having 2 to 6 carbon atoms or an alkoxycarbonyl group having 2 to 6 carbon atoms.

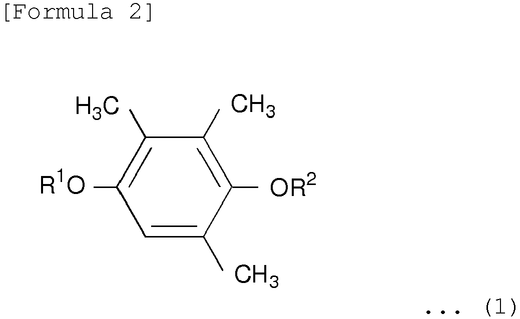

It is also preferred that this hydroquinone derivative is 2,3,5-trimethylhydroquinone-1-hexyl ether or 2,3,5-trimethylhydroquinone-1-hexyl ether 4-acetate. Thereby, a substance which is excellent in pharmacological activity and biocompatibility and can be used specifically effectively is selected.

Further, it is also preferred that the chronic respiratory disease is at least one disease selected from the group consisting of chronic obstructive pulmonary disease (COPD), interstitial pneumonia and asthma. Thereby, a suitable disease as a therapeutic target is selected. It is also preferred that, of these, the interstitial pneumonia is caused by an agent. The therapeutic agent of the present invention inhibits inflammation of lung tissue induced by an agent and effectively inhibits pulmonary fibrosis exhibited by interstitial pneumonia.

It is preferred that the agent described above is at least one agent selected from the group consisting of bleomycin, gefitinib, erlotinib, cetuximab, panitumumab, bortezomib, cisplatin, oxaliplatin, cyclophosphamide, azathioprine, tacrolimus, penicillamine, methotrexate, salazosulfapyridine, leflunomide, hydralazine, shosaikoto, amiodarone and interferon. Thereby, an appropriate agent which induces pulmonary fibrosis, i.e., interstitial pneumonia is selected.

The food composition for prevention or improvement of a chronic respiratory disease according to the present invention comprises, as an active ingredient, the hydroquinone derivative represented by the following general formula (1) wherein R¹ represents an alkyl group having 4 to 8 carbon atoms, and R² represents a hydrogen atom, an alkylcarbonyl group having 2 to 6 carbon atoms or an alkoxycarbonyl group having 2 to 6 carbon atoms.

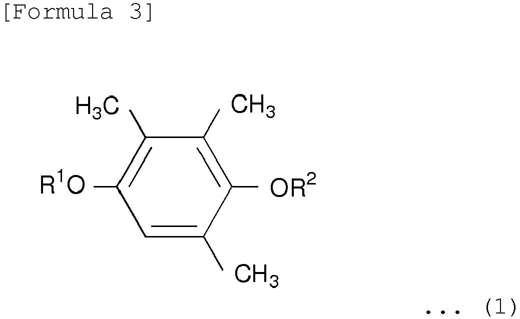

It is also preferred that the hydroquinone derivative, an active ingredient of the food composition for prevention or improvement of a chronic respiratory disease according to the present invention is 2,3,5-trimethylhydroquinone-1-hexyl ether or 2,3,5-trimethylhydroquinone-1-hexyl ether 4-acetate. Thereby, a substance which is excellent in pharmacological activity and biocompatibility and can be used specifically effectively is selected.

It is also preferred that the chronic respiratory disease is at least one disease selected from the group consisting of chronic obstructive pulmonary disease (COPD), interstitial pneumonia and asthma. Thereby, suitable pathological conditions to be prevented or improved are selected.

The composition for inhibiting cardiac fibrosis according to the present invention comprises, as an active ingredient, the hydroquinone derivative represented by the following general formula (1) wherein R¹ represents an alkyl group having 4 to 8 carbon atoms, and R² represents a hydrogen atom, an alkylcarbonyl group having 2 to 6 carbon atoms or an alkoxycarbonyl group having 2 to 6 carbon atoms.

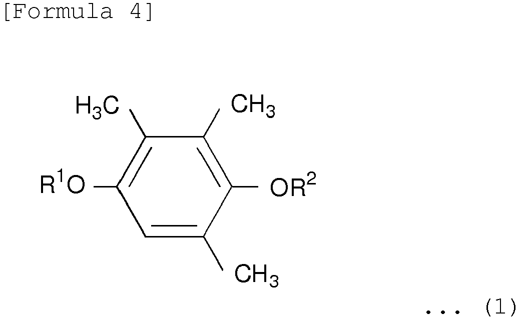

It is also preferred that this hydroquinone derivative is 2,3,5-trimethylhydroquinone-1-hexyl ether or 2,3,5-trimethylhydroquinone-1-hexyl ether 4-acetate. Thereby, a substance which is excellent in pharmacological activity and biocompatibility and can be used specifically effectively is selected.

It is also preferred that the cardiac fibrosis in the composition for inhibiting cardiac fibrosis according to the present invention is caused by an agent. The composition for inhibiting fibrosis according to the present invention inhibits inflammation of cardiac tissue induced by an agent and effectively inhibits cardiac fibrosis.

It is also preferred that the agent described above is an anthracyclin anticancer agent. Thereby, an appropriate agent which induces cardiac fibrosis is selected.

Further, the composition for alleviating a side effect of an agent according to the present invention comprises, as an active ingredient, the hydroquinone derivative represented by general formula (1) wherein R¹ represents an alkyl group having 4 to 8 carbon atoms, and R² represents a hydrogen atom, an alkylcarbonyl group having 2 to 6 carbon atoms or an alkoxycarbonyl group having 2 to 6 carbon atoms.

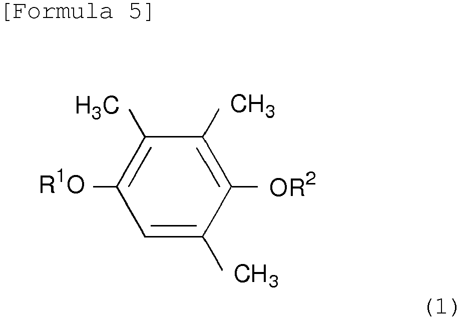

Further, it is preferred that the agent in the composition for alleviating a side effect of an agent according to the present invention is at least one agent selected from the group consisting of bleomycin, gefitinib, erlotinib, cetuximab, panitumumab, bortezomib, cisplatin, oxaliplatin, cyclophosphamide, azathioprine, tacrolimus, penicillamine, methotrexate, salazosulfapyridine, leflunomide, hydralazine, shosaikoto, amiodarone, interferon and an anthracyclin anticancer agent. The composition for alleviating a side effect according to the present invention inhibits inflammation of lung and cardiac tissue induced by these agents and effectively alleviates side effects such as myocardial disorder and interstitial pneumonia.

Further, it is also preferred that the agent in the composition for alleviating a side effect of an agent according to the present invention is bleomycin or an anthracyclin anticancer agent. The composition for alleviating a side effect according to the present invention inhibits inflammation of lung and cardiac tissue induced by these anticancer agents and effectively alleviates side effects such as myocardial disorder and interstitial pneumonia.

### Advantageous Effects of Invention

According to the present invention, a therapeutic agent for a chronic respiratory disease, a food composition for prevention or improvement of a chronic respiratory disease, a composition for inhibiting cardiac fibrosis and a composition for alleviating a side effect of an agent having excellent effects as follows can be provided.
(1) The progression of the condition of a chronic respiratory disease such as chronic obstructive pulmonary disease (COPD), asthma and interstitial pneumonia can be effectively inhibited and the pathological conditions can be improved by inhibiting inflammation of a respiratory tract and lung tissue and promoting the sputum excretion. Because the therapeutic agent for a chronic respiratory disease, the food composition for prevention or improvement of a chronic respiratory disease, the composition for inhibiting cardiac fibrosis and the composition for alleviating a side effect of an agent are consisted of highly safe substances, they can be effectively used for the prevention or treatment of these diseases.
(2) Inflammation of cardiac tissue can be inhibited and cardiac fibrosis diseases can be effectively inhibited. Because the therapeutic agent for a chronic respiratory disease, the food composition for prevention or improvement of a chronic respiratory disease, the composition for inhibiting cardiac fibrosis and the composition for alleviating a side effect of an agent are consisted of highly safe substances, they can be effectively used for the prevention or treatment of the disease.
(3) A composition which is excellent in pharmacological activity and biocompatibility and can be used specifically effectively can be obtained by selecting 2,3,5-trimethylhydroquinone-1-hexyl ether or 2,3,5-trimethylhydroquinone-1-hexyl ether 4-acetate.
(4) A therapeutic agent can be effectively administered, since the pulmonary or cardiac fibrosis induced by the therapeutic agent can be inhibited.
(5) An anticancer agent can be reliably administered, since the pulmonary or cardiac fibrosis induced by the anticancer agent such as bleomycin or anthracyclin anticancer agent can be inhibited and side effects such as interstitial pneumonia or myocardial disorder can be alleviated.

### Brief Description of Drawings

[Figure 1] Figure 1 is a graph showing the relative weights of lungs of rats in the control group and test groups (%) in Example 1.
[Figure 2] Figure 2 is a graph showing the total cell numbers in BAL fluid in the bronchoalveolar lavage examination in Example 1.
[Figure 3] Figure 3 is a graph showing the alveolar macrophage numbers in BAL fluid in the bronchoalveolar lavage examination in Example 1.
[Figure 4] Figure 4 is a graph showing the neutrophil numbers in BAL fluid in the bronchoalveolar examination lavage in Example 1.
[Figure 5] Figure 5 is a graph showing the lymphocyte numbers in BAL fluid in the bronchoalveolar examination lavage in Example 1.
[Figure 6] Figure 6 is a diagram showing the test flow of HTHQ-administered groups in Example 2.
[Figure 7] Figure 7 is a graph showing the inflammatory cell numbers in BAL fluid in the bronchoalveolar lavage examination in Example 2.
[Figure 8] Figure 8 is a graph showing the reactive oxygen species (ROS) levels in BAL fluid in the bronchoalveolar lavage examination in Example 2.
[Figure 9] Figure 9 is a graph showing the TNF-α levels in BAL fluid in the bronchoalveolar lavage examination in Example 2.
[Figure 10] Figure 10 is a graph showing the IL-6 levels in BAL fluid in the bronchoalveolar lavage examination in Example 2.
[Figure 11] Figure 11 is photos showing peribronchial lung tissue of the control group and test groups in Example 2.
[Figure 12] Figure 12 is a diagram showing the schedule of sensitization, causing diseases and administration of test materials in Example 3.
[Figure 13] Figure 13 is a graph showing the inflammatory cell numbers in BAL fluid in the bronchoalveolar lavage examination in Example 3.
[Figure 14] Figure 14 is a graph showing the IL-4 levels in BAL fluid in the bronchoalveolar lavage examination in Example 3.
[Figure 15] Figure 15 is a graph showing the IL-5 levels in BAL fluid in the bronchoalveolar lavage examination in Example 3.
[Figure 16] Figure 16 is a graph showing the IL-13 levels in BAL fluid in the bronchoalveolar lavage examination in Example 3.
[Figure 17] Figure 17 is a graph showing the total contents of IgE in the serums in Example 3.
[Figure 18] Figure 18 is a graph showing the contents of the ovalbumin-specific IgE in the serums in Example 3.
[Figure 19] Figure 19 is a graph showing the ability to excrete sputum of the control group and test groups in Example 4.

### Description of Embodiments

The present invention will be described in detail below.

The alkyl group having 4 to 8 carbon atoms represented by R¹ in the hydroquinone derivative represented by the general formula (1) may be linear, branched, or cyclic, and examples of the alkyl group include various butyl groups, various pentyl groups, various hexyl groups, various heptyl groups, various octyl groups, cyclobutyl groups, cyclopentyl groups, cyclohexyl groups, cycloheptyl groups and cyclooctyl groups. In terms of the pharmacological activity, this alkyl group is preferably a linear alkyl group having 4 to 7 carbon atoms and in particular an n-hexyl group is suitable.

The alkyl carbonyl group having 2 to 6 carbon atoms of R² may be linear or branched and examples of the alkyl carbonyl group include, for instance, acetyl groups, propionyl groups, butyryl groups and isobutyryl groups. Further, the alkoxycarbonyl group having 2 to 6 carbon atoms of R² may be linear or branched and examples of the alkoxycarbonyl group include, for instance, methoxycarbonyl groups, ethoxycarbonyl groups, propoxycarbonyl groups and isopropoxycarbonyl groups.

In terms of the pharmacological activity in any use, examples of the specifically preferred compound of the compounds represented by this general formula (1) can include 2,3,5-trimethylhydroquinone-1-butyl ether, 2,3,5-trimethylhydroquinone-1-hexyl ether and 2,3,5-trimethylhydroquinone-1-hexyl ether 4-acetate in any use.

The hydroquinone derivative represented by the general formula (1) can be manufactured by for example the method disclosed in Patent Literature 2.

The therapeutic agent for a chronic respiratory disease, the composition for inhibiting cardiac fibrosis and the composition for alleviating a side effect of an agent according to the present invention comprise the hydroquinone derivative represented by the general formula (1) as an active ingredient and have action which prevents or treats chronic respiratory disease such as chronic obstructive pulmonary disease (COPD), asthma and interstitial pneumonia, and inhibits cardiac fibrosis. Therefore, the therapeutic agent for a chronic respiratory disease, the composition for inhibiting cardiac fibrosis and the composition for alleviating a side effect of an agent according to the present invention can be used as a pharmaceutical agent, a quasi drug and a food composition for preventing, treating or improving these diseases. Examples of the causes of COPD, among chronic respiratory diseases, include toxic substance exposure, i.e., smoking (tobacco smoke), air pollution, inhalation of smoke of organic fuel and dust etc., and the symptoms can be improved by effectively inhibiting inflammation of a respiratory tract and lung tissue which can be caused by exposure of these toxic substance and promoting the sputum excretion at the same time according to the present invention. Meanwhile, interstitial pneumonia of the chronic respiratory diseases is caused by pulmonary fibrosis and examples of the causes of pulmonary fibrosis include, in addition to side effects of an agent, inhalation of powders of a mineral, powders of pottery or stone etc. and asbestos etc., radiation exposure, collagen diseases and infectious diseases etc. Examples of the causes of cardiac fibrosis include, in addition to side effects of an agent, viral infection, diabetes, obesity, thyroid diseases and alcohol etc. Pulmonary and cardiac fibrosis includes the pulmonary and cardiac fibrosis whose causes of onset have not been identified. According to the present invention, pulmonary or cardiac fibrosis caused by various causes is inhibited and in particular the pulmonary or cardiac fibrosis caused by an agent, i.e., the interstitial pneumonia or cardiomyopathy caused by an agent can be suitably inhibited. The type of the agent is not limited as long as it causes pulmonary or cardiac fibrosis and examples of the agent include an anticancer agent, an immunosuppressive agent, an antirheumatic drug, a vasodilator, an antiarrhythmic agent, a Kampo medicine, interferon, an antimicrobial agent, an antiepileptic drug, a diuretic or an antibiotic. Specifically, examples of the anticancer agent which causes cardiac fibrosis include an anthracyclin anticancer agent and examples of the anticancer agent which causes pulmonary fibrosis include bleomycin, gefitinib, erlotinib, cetuximab, panitumumab, bortezomib, vinorelbine, peplomycin, busulfan, irinotecan, cisplatin, oxaliplatin or carboplatin. Examples of the anthracyclin anticancer agent, among them, include doxorubicin (adriamycin), daunorubicin, pirarubicin, epirubicin, idarubicin, aclarubicin, amrubicin, valrubicin or mitoxantrone. Examples of the immunosuppressive agent which causes pulmonary fibrosis include cyclophosphamide, azathioprine, tacrolimus or penicillamine and examples of the antirheumatic drug which causes pulmonary fibrosis include methotrexate, salazosulfapyridine or leflunomide. While these agents are used to treat diseases such as malignant tumor and rheumatism, they cause pulmonary and cardiac fibrosis as a side effect. Therefore, side effects such as pulmonary or cardiac fibrosis can be decreased by taking the therapeutic agent for a chronic respiratory disease or the composition for inhibiting cardiac fibrosis according to the present invention before, simultaneously with, or sometime after the administration of these types of agents.

The dose of the therapeutic agent or composition of the present invention cannot be categorically defined because it varies depending on a target effect of prevention or treatment, a method of administration, an age and a body weight etc., and the parenteral dose per day is normally about 0.01 to 100 mg/kg body weight and is preferably about 0.05 to 50 mg/kg body weight in terms of the hydroquinone derivative described above. The dose of the therapeutic agent or composition of the present invention is orally about 0.1 to 500 mg/kg body weight and is preferably about 0.5 to 200 mg/kg body weight in terms of the hydroquinone derivative described above, and these doses can be divided into 1 to 3 portions to administer. When the therapeutic agent or composition of the present invention is used to inhibit the pulmonary and cardiac fibrosis caused by other agents, the therapeutic agent or composition of the present invention is preferably administered before the administration of the agents such as an anticancer agent which causes the pulmonary and cardiac fibrosis, or they can be administered simultaneously with or separately from the administration of the agents.

To simultaneously administer an agent such as the anticancer agent and the immunosuppressive agent which cause pulmonary and cardiac fibrosis and the hydroquinone derivative described above, an active ingredient of the therapeutic agent or composition of the present invention, a combination drug in which such an agent which causes pulmonary and cardiac fibrosis and the hydroquinone derivative are combined can be used.

Further, the therapeutic agent for a chronic respiratory disease, the composition for inhibiting cardiac fibrosis and the composition for alleviating a side effect of an agent according to the present invention can contain genetically modified human erythropoietin (EPO) in addition to the hydroquinone derivative represented by the general formula (1) described above. Hereby, a more improved fibrosis inhibiting effect and inflammation inhibiting effect can be obtained. The dose in terms of human erythropoietin combined with the hydroquinone derivative described above cannot be categorically defined because it varies depending on a target therapeutic effect, a method of administration, an age and a body weight etc. and the parenteral dose per day is normally about 0.1 to 100 IU/kg body weight, and is preferably about 0.5 to 50 IU/kg body weight. The oral dose is about 1 to 1000 IU/kg body weight and is preferably about 5 to 500 IU/kg body weight, and these doses can be divided into 1 to 3 portions to administer. These active ingredients in the composition or therapeutic agent in which the hydroquinone derivative described above and the human erythropoietin described above are combined can be administered separately or simultaneously, orally or parenterally as a pharmaceutical composition. When the hydroquinone derivative and the human erythropoietin, active ingredients, are formulated separately, the separately formulated formulations can be mixed to administer at the time of use, or the separately formulated formulations can be administered separately, simultaneously or after sometime to the same subject.

The therapeutic agent for a chronic respiratory disease, the composition for inhibiting cardiac fibrosis and the composition for alleviating a side effect of an agent according to the present invention can be prepared in various forms by conventionally widely used methods. In this case, those can be formulated with an excipient which is accepted as the excipient of a pharmaceutical agent such as a carrier or a vehicle for a standard formulation. To improve the bioavailability and stability of the present compound, a drug delivery system including a formulation technique such as microcapsule, micronization and clathration using cyclodextrin etc. can be used.

When the composition is used as a formulation for oral administration, the composition can be used in a form such as a tablet, a granule, a capsule or a liquid for oral administration, but it is preferably used in a form suitable for adsorption from a gastrointestinal tract. A conventional formulation technique can be used also when the formulation is provided in a desired form in terms of distributivity and preservability. When the composition is used as an agent for parenteral administration, the formulation can be in the form of an injection, a suppository and percutaneous absorption agent etc. such as a tape and a cataplasm, or can be used after dissolving a solid formulation in an appropriate solvent at the time of use for the sake of distributivity and preservability, or can be provided in a form of a liquid or a semisolid formulation according to a conventional formulation technique.

The food composition for prevention/improvement of a chronic respiratory disease, cardiac fibrosis diseases or side effects of an agent comprising the hydroquinone derivative represented by the general formula (1) described above as an active ingredient can be used in any form including a form of a supplement such as a tablet, a capsule, a granule and a syrup, a beverage, confectionery, a bread, rice gruel, a cereal, a noodle, a jelly, a soup, a dairy product, a flavoring and an edible oil. When the composition is used as a food composition, other active ingredients, nutrients etc. such as a vitamin, a mineral or an amino acid etc. can be variously combined with the composition to the extent that they do not affect the potency of the active ingredient of the present invention. The foods obtained from the food composition of the present invention include a supplement, a health food, a functional food and a specified health food etc. The amount of intake of the food composition of the present invention is preferably about 0.1 to 500 mg/kg body weight and is more preferably about 0.5 to 200 mg/kg body weight in terms of the hydroquinone derivative described above, and the amount is preferably divided into 1 to 3 portions to take.

Now, the present invention will be described in more detail by way of Examples, but the present invention is not limited by these Examples in any way.

### Example

### [Example 1]

### 1. Study of the action on pulmonary fibrosis induced by bleomycin

Bleomycin is used to produce disease-model animals of interstitial pneumonia. The bleomycin and 2,3,5-trimethylhydroquinone-1-hexyl ether (HTHQ) as the hydroquinone derivative represented by the general formula (1) described above of the present invention were simultaneously administered to male SD rats of 10 week old after birth to examine the effect of the action. The test groups consisted of the control group to which sterile saline was administered; test group 1 to which bleomycin (7.5 mg/kg body weight) alone was administered; test group 2 to which bleomycin (7.5 mg/kg body weight) and HTHQ (50 mg/kg body weight/day) were administered in combination; and test group 3 to which bleomycin (7.5 mg/kg body weight) and HTHQ (200 mg/kg body weight/day) were administered in combination.

In test groups 1 to 3, bleomycin was orally administered in a single dose. In test groups 2 and 3, 2,3,5-trimethylhydroquinone-1-hexyl ether (HTHQ) was daily orally administered for 10 or 20 days starting from 24 h after the administration of bleomycin. In test group 1, olive oil, which was used as a solvent of HTHQ, was daily orally administered at 10 mL/kg/day starting from 24 h after the administration of bleomycin. The number of animals in each group was 16. 8 animals of each group were sacrificed on days 10 and 20 after the administration of bleomycin and measurement of body weight, lung autopsy, histopathologic examination of lungs and bronchoalveolar lavage examination were conducted.

### <Body weight and relative weight of lungs>

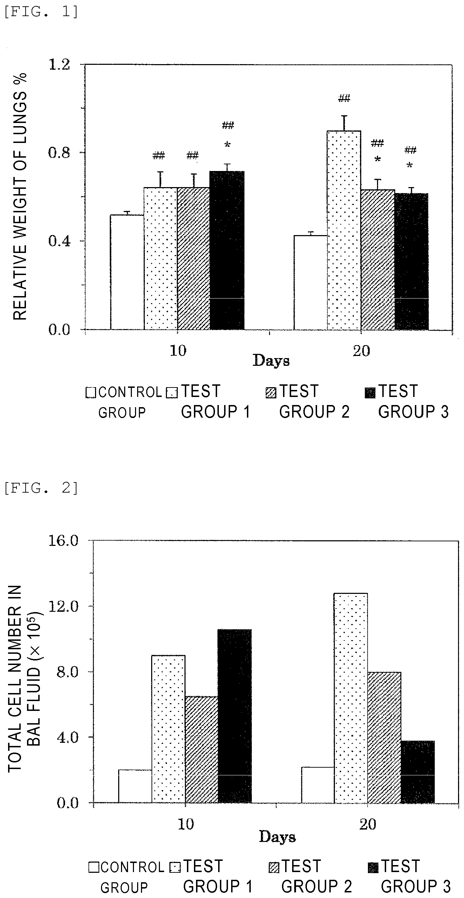

The body weight of the rats in the control group increased over time during the test period. On the other hand, the body weight of the rats in test groups 1 to 3, to which bleomycin was administered, gradually decreased during the test period. Here, the result of the relative weight of lungs is shown in Figure 1. The weight of the interstitial tissue of lungs tends to increase and the relative weight of lungs tends to rise when the tissue fibroses. The number in the graph indicates the corresponding test group, and ## described above bars indicates that the p value is < 0.01 in comparison to the control group, and * indicates that p value is < 0.05 in comparison to test group 1. As shown in Figure 1, the relative weight of lungs of test groups 1 to 3, to which bleomycin was administered, significantly increased compared to the control group (p < 0.01). However, the relative weight of lungs of test groups 2 and 3, to which HTHQ was administered, on day 20 after the administration was significantly low compared to the bleomycin-single administration group (test group 1) (p < 0.05). Thus, it was expected that the degree of the progression of pulmonary fibrosis of the rats in test groups 2 and 3, to which HTHQ was administered, was less than the rats in test group 1.

### <Gross pathology of lungs>

The lung autopsy was conducted and the gross pathology was observed as follows: delomorphous nodules as well as many dark red and light red ecchymoses were observed mainly in the hilar area and the surfaces of lungs were depressed in the lungs of the rats in test group 1 (the bleomycin-single administration group) on day 20 after the administration. On the other hand, such lesions were alleviated in the lungs of the rats in test groups 2 and 3, to which HTHQ was administered.

### <Findings from the histopathologic examination of lungs>

The main lung lesions observed in the rats in test group 1 (the bleomycin-single administration group) on day 10 after the administration were the peribronchial and peribronchiola enlargement of alveoli, the hyperplasia of the alveolar wall, the infiltration of monocytes and lymphocytes in alveolar walls and interstitial tissue, and the exudation of alveolar macrophage into alveolar spaces. These lesions were alleviated in rats in test groups 2 and 3 (the HTHQ-administered groups) compared to the tissue in test group 1. Further, in test group 1, it was proved that pulmonary fibrosis has progressed because atypias of alveolar epitheliums having large nuclei whose nucleoli are not clear were found and foamy alveolar macrophages were observed within alveolar spaces of the sites in which the morphology of alveolus was still maintained.

The result of the histopathologic examination of lungs of the control group and the test groups on day 20 after the administration of bleomycin is shown in Table 1 below. "-" indicates "not found", "+" indicates "mild", "++" indicates "moderate", and "+++" indicates "severe" in severity scores. In test group 1 (the bleomycin-single administration group), the hyperplasia and fibrosis of peribronchial and adjacent alveolar walls became remarkable compared to the tissue on day 10 after the administration and the morphology of alveolus almost disappeared due to the infiltration of lymphocytes and neutrophils. The alveolar macrophages infiltrated within alveolar spaces were buried in surrounding tissues. On the other hand, in test groups 2 and 3, the HTHQ-administered groups, though the alveolar macrophages containing vacuoles, the cell infiltration and the hyperplasia of peribronchiolar alveolar walls were found within alveolar spaces of the sites in which the morphology of alveolus was still maintained, the degree of these lesions was milder than test group 1, the bleomycin-single administration group.

**[Table 1]**

| Lesion | | Severity score | | | |
|---|---|---|---|---|---|
| | | Control group | Test group 1 | Test group 2 | Test group 3 |
| Type II alveolar epithelial cell | Atypia and dysplasia | - | ++ | + | + |
| | Adenomatous hyperplasia | - | ++ | ++ | + |
| Alveolar space | Bleeding | - | + | + | - |
| | Protein exudation | - | +++ | + | ++ |
| | Macrophage accumulation | - | +++ | + | + |
| Alveolar wall | Interstitial edema | - | + | + | + |
| | Mononuclear cell infiltration | - | +++ | ++ | ++ |
| | Fibroblast accumulation | - | +++ | ++ | + |
| | Fibrosis of interstitial tissue | - | +++ | ++ | + |
| Circumvascular region | Perivascular edema | - | + | + | - |
| | Plasma cell infiltration | - | + | + | - |
| Peribronchial and peribronchiolar regions | | | | | |
| | Fibrosis of interstitial tissue | - | +++ | + | + |
| | Lymphoid follicle hyperplasia | - | ++ | + | + |

### <Bronchoalveolar lavage (BAL) examination>

Bronchoalveolar lavage (BAL) examination was conducted on the rats in the control group and the test groups. The measurement result of the total cell numbers within bronchoalveolar lavage fluid is shown in Figure 2. When interstitial pneumonia developed, the infiltration of inflammatory cells occurs and thus the total cell number in BAL fluid increases. The total cell numbers on day 10 after the administration of bleomycin were as follows: 2 × 10⁵ cells in the control group, 9 × 10⁵ cells in test group 1 (the bleomycin-single administration group), 6.5 × 10⁵ cells in test group 2 (the low-dose HTHQ-administered group, 50 mg/kg body weight), and 10.5 × 10⁵ cells in test group 3 (the high-dose HTHQ-administered group, 200 mg/kg body weight), and thus the effect of HTHQ administration was not found on day 10 after the administration of bleomycin. However, on day 20 after the administration of bleomycin, the total cell number of the bleomycin-single administration group was 12.8 × 10⁵ cells and the total cell number of the low-dose HTHQ-administered group was 7.9 × 10⁵ cells, and the total cell number of the high-dose HTHQ-administered group was reduced to 3.8 × 10⁵ cells. Therefore, it was proved that HTHQ inhibits the exudation of lung cells in a dose-dependent manner.

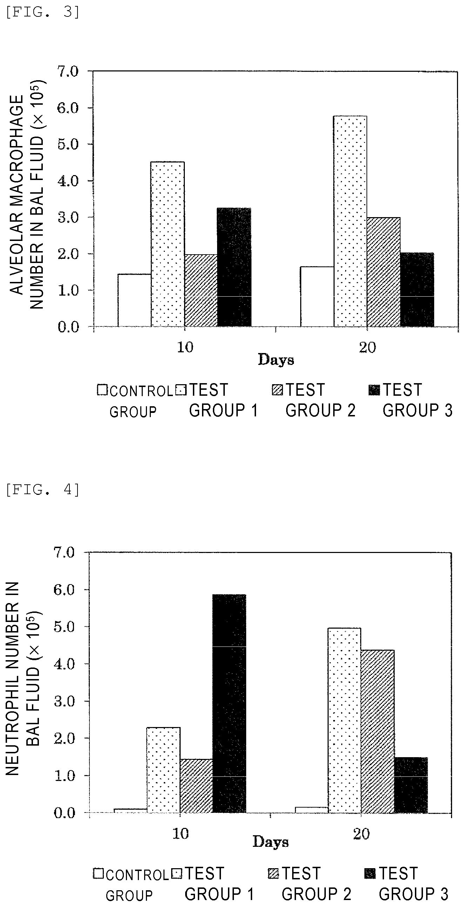

The measurement result of the alveolar macrophage numbers within bronchoalveolar lavage fluid is shown in Figure 3. When interstitial pneumonia developed, the infiltration of alveolar macrophages to interstitial tissue occurs and thus the alveolar macrophage number in BAL fluid increases. The alveolar macrophage numbers on day 10 after the administration of bleomycin were as follows: 1.45 × 10⁵ cells in the control group, 4.5 × 10⁵ cells in test group 1 (the bleomycin-single administration group), 1.99 × 10⁵ cells in test group 2 (the low-dose HTHQ-administered group), and 3.24 × 10⁵ cells in test group 3 (the high-dose HTHQ-administered group), and thus the effect of HTHQ administration was not found on day 10 after the administration of bleomycin. However, on day 20 after the administration, while the alveolar macrophage number of the bleomycin-single administration group was 5.5 × 10⁵ cells, the alveolar macrophage number of the low-dose HTHQ-administered group was 3 × 10⁵ cells and the alveolar macrophage number of the high-dose HTHQ-administered group was decreased to 2 × 10⁵ cells. Therefore, it was proved that HTHQ inhibits the exudation of alveolar macrophages in a dose-dependent manner.

Further, the measurement result of the neutrophil numbers within bronchoalveolar lavage fluid is shown in Figure 4. When interstitial pneumonia developed, the infiltration of inflammatory cells of neutrophils to interstitial tissue occurs and thus the neutrophil number in BAL fluid increases. The neutrophil numbers on day 10 after the administration of bleomycin were as follows: 1.04 × 10⁴ cells in the control group, 2.28 × 10⁵ cells in test group 1 (the bleomycin-single administration group), 1.44 × 10⁵ cells in test group 2 (the low-dose HTHQ-administered group), and 5.83 × 10⁵ cells in test group 3 (the high-dose HTHQ-administered group), and thus the effect of HTHQ was not found on day 10 after the administration of bleomycin. However, on day 20 after the administration, while the neutrophil number of the bleomycin-single administration group was 4.97 × 10⁵ cells, the neutrophil number of the low-dose HTHQ-administered group was 4.34 × 10⁵ cells and the neutrophil number of the high-dose HTHQ-administered group was significantly reduced to 1.49 × 10⁵ cells. Therefore, it was proved that the administration of high-dose HTHQ can significantly reduce the neutrophil numbers.

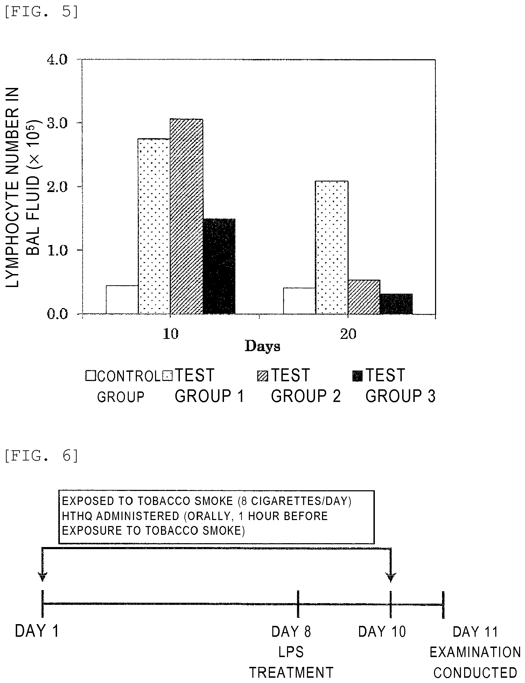

Then, the measurement result of the lymphocyte numbers within bronchoalveolar lavage fluid is shown in Figure 5. When interstitial pneumonia developed, the infiltration of inflammatory cells of lymphocytes to interstitial tissue occurs, and thus the lymphocyte numbers in BAL fluid increases. The lymphocyte numbers on day 10 after the administration of bleomycin were as follows: 4.42 × 10⁴ cells in the control group, 2.72 × 10⁵ cells in test group 1 (the bleomycin-single administration group), 3.07 × 10⁵ cells in test group 2 (the low-dose HTHQ-administered group), and 1.43 × 10⁵ cells in test group 3 (the high-dose HTHQ-administered group), and thus the effect of HTHQ administration was not found on day 10 after the administration. However, on day 20 after the administration, while the lymphocyte number in bleomycin-single administration group was 2.09 × 10⁵ cells, the lymphocyte number of the low-dose HTHQ-administered group was 0.53 × 10⁵ cells and the lymphocyte number of high-dose HTHQ-administered group was significantly reduced to 0.3 × 10⁵ cells. Therefore, it was proved that HTHQ inhibits the exudation of lymphocyte in a dose-dependent manner.

The results of the histopathologic examination, bronchoalveolar lavage examination, etc. proved that the hydroquinone derivative represented by the general formula (1) described above of the present invention has the effect to effectively inhibit pulmonary fibrosis induced by bleomycin and prevent or treat interstitial pneumonia.

### [Example 2]

### 2. Study of the action on lung inflammation induced by tobacco smoke

6 week old male SPF C57BL/6N mice, 20 to 25 g of body weight, were purchased from CORETEC INC. (South Korea). After quarantine and adaptation period of about 1 week, mice were divided into 5 groups shown in Table 2 below.

**[Table 2]**

| Control group and test groups | | Details of administration | | |
|---|---|---|---|---|
| Control group | Normal control group "NC" | No agent administered | Not exposed to tobacco smoke | LPS not administered |
| Test group | COPD model group "COPD" | No agent administered | Exposed to tobacco smoke | LPS administered |
| | Positive control su bstance-ad mi nistered group "ROF" | Roflumilast administered at 10 mg/kg/day | | |
| | HTHQ-administered group (low-dose) "HTHQ10" | HTHQ administered at 10 mg/kg/day | | |
| | HTHQ-administered group (high-dose) "HTHQ20" | HTHQ administered at 20 mg/kg/day | | |

The tests were conducted as follows. The mice in the COPD (chronic obstructive pulmonary disease) model group of the test groups were exposed to tobacco smoke for 1 hour per day (8 cigarettes/day) for 10 days and LPS (5 µg/50 µL/mouse) was intranasally administered to the mice on day 8 after the start of the tests. Roflumilast, the positive control substance, was orally administered to the mice in the positive control substance-administered group at 10 mg/kg body weight/day, and then they were exposed to tobacco smoke for 10 days starting from 1 hour after the administration of roflumilast, and LPS (5 µg/50 µL/mouse) was intranasally administered to the mice on day 8 after the start of the tests. Here, roflumilast is a selective phosphodiesterase 4 inhibitor and is a substance which is used as a therapeutic agent of COPD and asthma (approved in Europe and the United States, not approved in Japan). Meanwhile, as shown in Figure 6, 2,3,5-trimethylhydroquinone-1-hexyl ether (HTHQ), the test substance was orally administered to the mice in the HTHQ-administered groups at 10 mg/kg body weight/day for the low-dose group and at 20 mg/kg body weight/day for the high-dose group, respectively, and then they were exposed to tobacco smoke for 10 days starting from 1 hour after the administration of the test substance, and LPS (5 µg/50 µL/mouse) was intranasally administered to the mice on day 8. All the animals were euthanized on day 11 and bronchoalveolar lavage (BAL) examination and histopathologic examination of lungs were conducted. The mice in the normal control group and the test groups were fed sterile tap water and standard food for rodents during the test period. All experimental procedures were conducted after receiving the IACUC approval of Korea Research Institute of Bioscience and Biotechnology.

### <Bronchoalveolar lavage (BAL) examination>

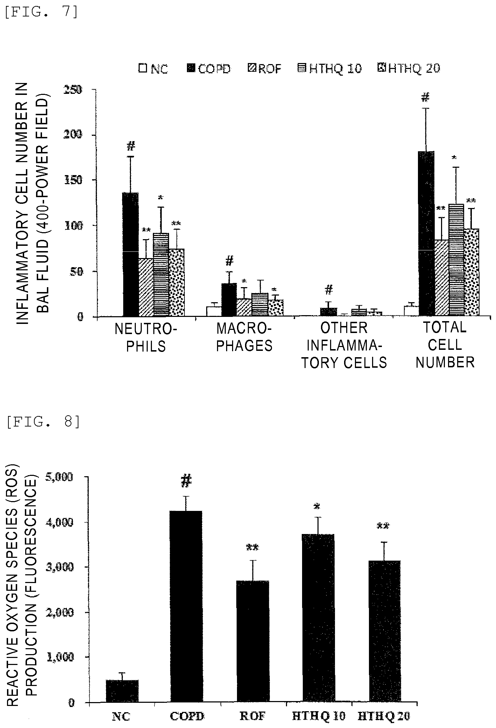

The mice in the test groups were euthanized 72 hours after the LPS administration and the mice in the normal control group were euthanized on day 11 after the start of the tests by intraperitoneal injection of pentobarbital (Hanrimu pharmaceutical, South Korea) at 50 mg/kg and then the bronchi were excised. To collect bronchoalveolar lavage fluid (BAL fluid), 700 µL of ice-cold PBS was injected to the lungs and recovered, and this process was repeated twice to collect 1.4 mL of BAL fluid. The collected BAL fluid was centrifuged at 4°C, 1500 rpm × 5 min. The supernatant was collected and stored in a super-cryostat at -70°C for later pro-inflammatory cytokine analysis (TNF-α and IL-6). Meanwhile, 1 mL of PBS was injected to the cells precipitated by the centrifugation and the mixture was tapped and suspended to obtain BAL cell fluid. After preparing slide samples from 100 µL of BAL cell fluid using Cytospin (4°C, 1000 rpm × 5 min), the cell numbers of inflammatory cells (neutrophil, macrophages) present in the BAL fluid were counted using a Diff-Quik stain kit. The result is shown in Figure 7. As shown in Figure 7, infiltration of inflammatory cells was recognized in the mice in the COPD model group induced by tobacco smoke. On the other hand, infiltration of inflammatory cells was effectively reduced in the mice in the HTHQ-administered groups compared to the COPD model group. The difference of potency between the doses of HTHQ was not observed and the administration of HTHQ exhibited potency similar to the administration of roflumilast (ROF), the positive control substance.

Reactive oxygen species (ROS) amounts in BAL cell fluid were measured. BAL cell fluid was added to wells of a 96 well plate at 5 × 10³/100 µL/well, and then 10 µL aliquots of 20 mM of DCF-DA, as a ROS indicator, were added to the wells, and the mixtures were shaken for 30 minutes. Reactive oxygen species (ROS) amounts within cells were measured at an excitation wavelength of 485 nm and a fluorescence wavelength of 530 nm using a fluorescence plate analyzer (a product from PerkinElmer, Inc.). The result is shown in Figure 8. As shown in Figure 8, high production of reactive oxygen species was found in the mice in COPD model group induced by tobacco smoke. On the other hand, the amount of reactive oxygen species in the mice in the HTHQ-administered groups was effectively reduced compared to the mice in the COPD group, and HTHQ exhibited potency almost similar to the positive control substance in the comparison to the positive control substance (roflumilast, ROF) administered group.

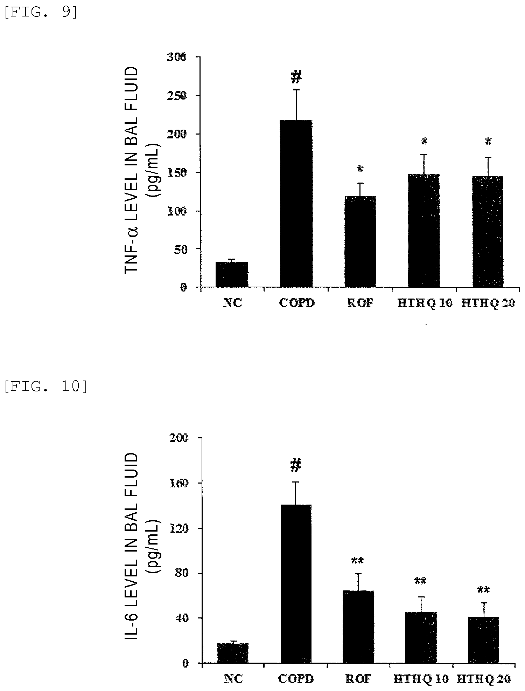

The supernatant of the BAL fluid stored was taken out of the super-cryostat, and TNF-α and IL-6 levels were measured as the levels of pro-inflammatory cytokines in the supernatant. A quantitative ELISA kit (a product from Invitrogen) and an ELISA analyzer (a product from Molecular Devices, LLC.) were used for the measurement and the measurement wavelength was 450 nm. The result of TNF-α levels is shown in Figure 9 and the result of IL-6 levels is shown in Figure 10. As shown in Figure 9, it was proved that the BAL fluid of the COPD model group contained a high level of TNF-α. On the other hand, TNF-α levels of the HTHQ-administered groups were effectively reduced compared to the COPD model group, and HTHQ exhibited potency almost similar to the positive control substance in the comparison to the positive control substance (roflumilast, ROF) administered group. About IL-6, as shown in Figure 10, the BAL fluid of the COPD model group contained a very high level of IL-6. The IL-6 levels of the HTHQ-administered groups were significantly reduced compared to that of the COPD model group, and thus it was proved that HTHQ exhibited potency similar to ROF (roflumilast).

### <Histopathologic examination of lungs>

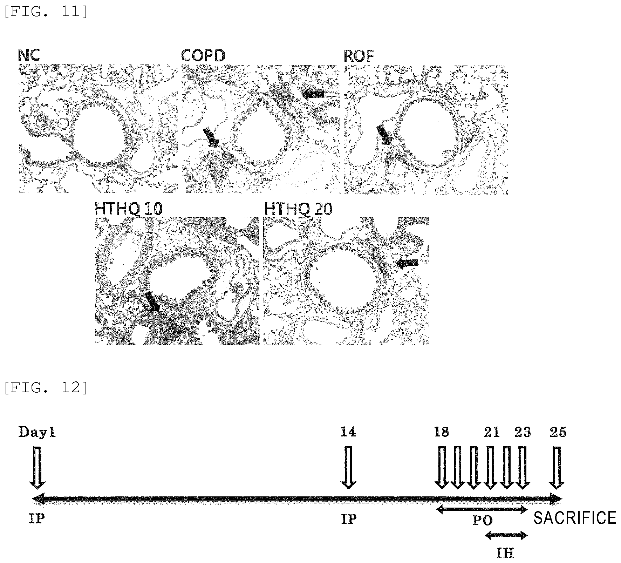

Bronchoalveolar lavage (BAL fluid) of the mice in the normal control group and test groups was collected and then the peribronchial lung tissues were fixed with a 10% neutral formalin solution. The lung tissues were embedded in paraffin and then sliced to 4 µm thick sections, and the sections were subjected to hematoxylin-eosin stain and observed. The photos of the lung tissues around the respiratory of the normal control group ("NC" in the photos) and the test groups are shown in Figure 11. In the photos of Figure 11, the parts in which the infiltration of inflammatory cells is occurring (the parts densely stained by HE stain) are indicated by arrows. As shown in the photos of Figure 11, extensive infiltration of many inflammatory cells was observed in the mice in the COPD model group ("COPD" in the photos) induced by tobacco smoke. On the other hand, infiltration of inflammatory cells was significantly reduced in a dose-dependent manner in the HTHQ-administered groups ("HTHQ10" and "HTHQ20" in the photos) compared to the COPD model group. HTHQ exhibited potency similar to roflumilast in the comparison to the roflumilast, which was used as the positive control substance, administered group ("ROF" in the photos).

These results of bronchoalveolar lavage examination and histopathologic examination proved that the hydroquinone derivative represented by the general formula (1) described above of the present invention is effective in effectively inhibiting inflammation of lung tissue induced by tobacco smoke, i.e., progression of COPD and in preventing or treating COPD. The hydroquinone derivative of the present invention exhibited potency similar to roflumilast used as the positive control substance in the Examples, and thus it was shown that the hydroquinone derivative is effective in the prevention and treatment of COPD.

### [Example 3]

### 3. Study of action on asthma

The efficacy of the hydroquinone derivative represented by the general formula (1) described above of the present invention on asthma was studied using an allergic asthma model of mice caused by ovalbumin sensitization.

6 week old BALB/c female mice were purchased and the mice were divided into 5 groups with 5 animals per group shown in Table 3 below after habituation breeding of about 2 weeks.

**[Table 3]**

| Control group and test groups | | Details of administration |
|---|---|---|
| Control group | Normal control group "NC" | No agent administered |
| Test group | Ovalbumin-sensitized control group "OVA" | No agent administered |
| | Positive control substance-administered group "Mon" | Montelukast administered at 30 mg/kg/day |
| | HTHQ-administered group (low-dose) "HTHQ20" | HTHQ administered at 20 mg/kg/day |
| | HTHQ-administered group (high-dose) "HTHQ40" | HTHQ administered at 40 mg/kg/day |

The test was conducted on the schedule of sensitization, causing diseases and administration of test materials shown in Figure 12. "IP" in Figure 12 refers to ovalbumin sensitization treatment by the intraperitoneal administration of ovalbumin/aluminum hydroxide, and "IH" refers to inhalation exposure treatment of ovalbumin, and "PO" refers to the administration of the test materials. Specifically, 200 µL of PBS (pH 7.4) emulsified by the addition of 20 µg ovalbumin and 2 mg aluminum hydroxide as an adjuvant was intraperitoneally administered to all mice in 4 groups except the normal control group (the initial sensitization, day 1 of the test). Then, after 2 weeks (day 14 of the test), the second sensitization treatment was conducted in the same way as the initial sensitization. Further, the mice were subjected to inhalation exposure of 1% ovalbumin-containing PBS of 1 hour per day on days 21 to 23 of the test using an ultrasonic nebulizer. Meanwhile, the test materials were daily orally administered to the mice on days 18 to 23 of the test. Specifically, PBS was orally administered to the normal control group, and 3% Tween 80-containing saline below which was used as a solvent of HTHQ was orally administered to the ovalbumin sensitization control group, and montelukast (a product from Sigma-Aldrich) dissolved in PBS was orally administered to the positive control substance-administered group at 30 mg/kg body weight/day. Here, montelukast is a leukotriene receptor antagonist and is a substance which is used as a therapeutic agent of bronchial asthma. 2,3,5-trimethylhydroquinone-1-hexyl ether (HTHQ), the test substance, dissolved in 3% Tween 80 was orally administered to the low-dose group of the HTHQ-administered groups at 20 mg/kg body weight/day and to the high-dose group of the HTHQ-administered groups at 40 mg/kg body weight/day, respectively. On day 25 of the test, blood was collected from orbital sinuses of all mice, and then the mice were euthanized and bronchoalveolar lavage (BAL) examination and measurement of the total contents of IgE and the contents of ovalbumin-specific IgE in the serums were conducted.

### <Bronchoalveolar lavage (BAL) examination>

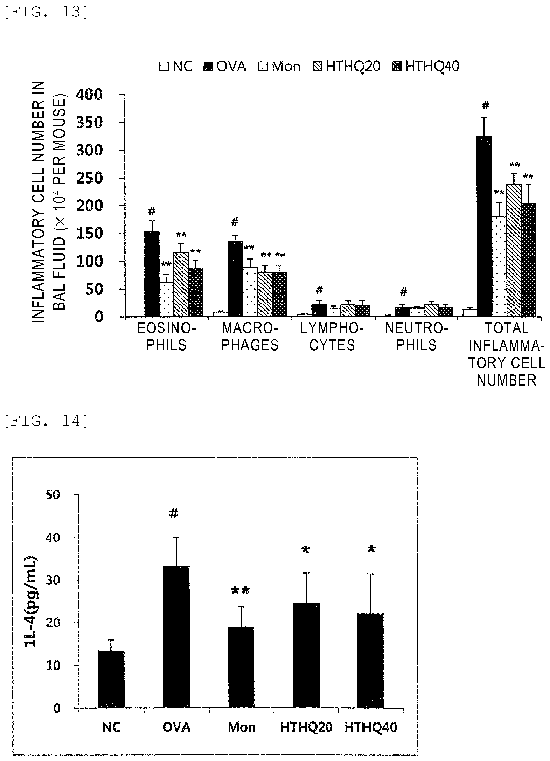

All mice were euthanized by intraperitoneal injection of pentobarbital (Hanrimu pharmaceutical, South Korea) at 50 mg/kg and the bronchi were excised on day 25 of the test. To collect bronchoalveolar lavage fluid (BAL fluid), 700 µL of ice-cold PBS was injected to the lungs and recovered, and this process was repeated twice to collect 1.4 mL of BAL fluid. The collected BAL fluid was centrifuged at 4°C, 1500 rpm × 5 min. The supernatant was collected and stored in a super-cryostat at -70°C for later pro-inflammatory cytokine analysis. 1 mL of PBS was injected to the cells precipitated by the centrifugation and the mixture was tapped and suspended to obtain BAL cell fluid. After preparing slide samples from 100 µL of BAL cell fluid using Cytospin (4°C, 1000 rpm × 5 min), the cell numbers of inflammatory cells (eosinophils, macrophages, lymphocytes and neutrophils) present in the BAL fluid were counted using a Diff-Quik stain kit. The result is shown in Figure 13. # described above bars in the graph of Figure 13 indicates that p value is < 0.01 in comparison to the normal control group, and ** indicates that p value is < 0.01 in comparison to the ovalbumin sensitization control group. As shown in Figure 13, the total number of eosinophils, macrophages and inflammatory cells was increased in the mice in the ovalbumin sensitization control group "OVA" sensitized by intraperitoneal administration of ovalbumin and having allergic asthma caused by inhalation of ovalbumin compared to the mice in the normal control group "NC". In contrast, the inflammatory cell numbers described above were significantly reduced in an HTHQ dose-dependent manner in the mice in the HTHQ-administered groups, and thus it was proved that the inhibitory action of inflammatory cell numbers of HTHQ was comparable to that of the positive control substance, montelukast, which is used as a therapeutic agent of bronchial asthma (refer to the positive control substance-administered group "Mon") .

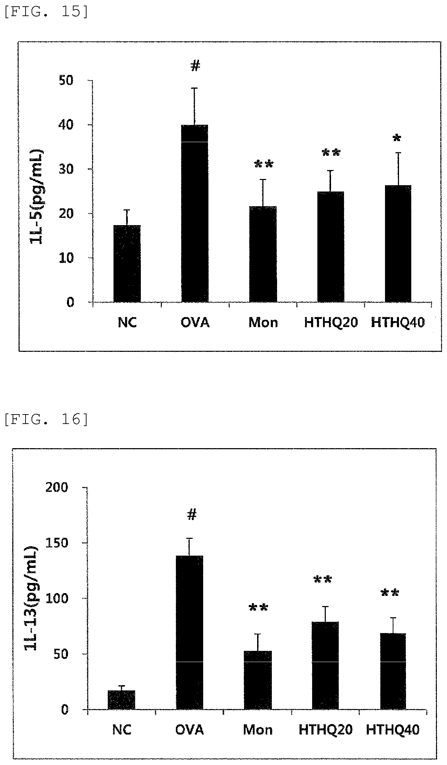

The supernatant of the BAL fluid stored was taken out of the super-cryostat and IL-4, IL-5 and IL-13 levels were measured as the pro-inflammatory cytokine contents in the supernatant of the BAL fluid. A quantitative ELISA kit (a product from R&D systems) and a microplate reader (a product from Bio-Rad Laboratories, Inc.) were used for the measurement and the measurement wavelength was 450 nm. The results of IL-4, IL-5 and IL-13 are shown in Figure 14, 15 and 16, respectively. # described above bars in the graphs of Figures 14 to 16 indicates that p value is < 0.01 in comparison to the normal control group, and * indicates that p value is < 0.05 in comparison to the ovalbumin sensitization control group, and ** indicates that p value is < 0.01 in comparison to the ovalbumin sensitization control group. As shown in Figures 14 to 16, the contents of IL-4, IL-5 and IL-13 were significantly increased in the BAL fluid of the ovalbumin sensitization control group "OVA" compared to those of the normal control group "NC". In contrast, it was shown that the amounts of these cytokines were significantly reduced in the HTHQ-administered groups compared to those of the ovalbumin sensitization control group "OVA", and the similar significant reduction was found also in the positive control substance-administered group "Mon".

### <Total contents of IgE and contents of ovalbumin-specific IgE in serums>

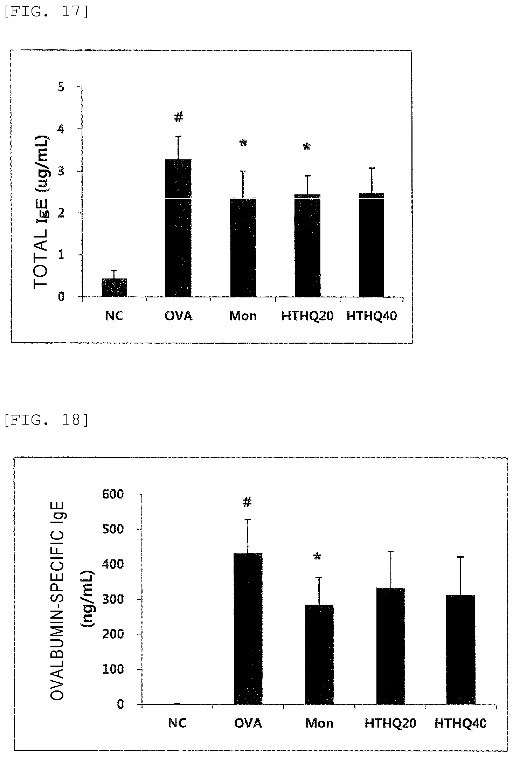

On day 25 of the test, the total contents of IgE and contents of ovalbumin-specific IgE in serums were measured using blood collected from orbital sinuses of the mice. An ELISA kit for IgE measurement (a product from BioLegend, Inc.) was used for the measurement and the contents were measured at wavelength of 450 nm using a micro plate reader (a product from Bio-Rad Laboratories, Inc.). The result of the total contents of IgE in the serums is shown in Figure 17 and the result of the contents of ovalbumin-specific IgE in serums is shown in Figure 18. # described above bars in the graphs of Figure 17 and 18 indicates that p value is < 0.01 in comparison to the normal control group, and * indicates that p value is < 0.05 in comparison to the ovalbumin sensitization control group. As shown in Figure 17 and 18, the total amount of IgE and the amount of ovalbumin-specific IgE in serum in the ovalbumin sensitization control group "OVA" was clearly increased compared to those of the normal control group "NC". In contrast, in the HTHQ-administered groups, significant reduction of the total amount of IgE was found in the 20 mg of HTHQ/kg body weight/day-administered group "HTHQ20" and a declining trend was found in the 40 mg of HTHQ/kg body weight/day-administered group "HTHQ40", as shown in Figure 17. On the other hand, the significant difference of the amounts of ovalbumin-specific IgE resulting from HTHQ administration was not found, but a declining trend was seen, as shown in Figure 18.

The above mentioned results proved that HTHQ has the action comparable to montelukast, a leukotriene receptor antagonist, which has been already clinically used as a therapeutic agent of bronchial asthma, i.e., the action to effectively inhibit the inflammation of respiratory tracts caused by allergic reaction. Therefore, it was shown that the hydroquinone derivative represented by the general formula (1) described above of the present invention is effective in the treatment of bronchial asthma.

### [Example 4]

### 4. Study of sputum excretion action

The hydroquinone derivative of the present invention was orally administered to 8 week old male ICR mice in a single dose, and the sputum excretion action was evaluated according to the method of Engler et al. (Engler H, Szelenyi I, J.Pharmacol.Moth.11, 151-157, 1984). First, the 8 week old ICR male mice were divided into 5 groups with 8 animals per group shown in Table 4 below.

**[Table 4]**

| Control group and test groups | Details of administration |
|---|---|
| Negative control | Aqueous solution of 2% gum arabic administered |
| HTHQ 100 mg/kg | HTHQ administered at 100 mg/kg |
| HTHQ 200 mg/kg | HTHQ administered at 200 mg/kg |
| HTHQ 400 mg/kg | HTHQ administered at 400 mg/kg |
| Ambroxol 250 mg/kg | Ambroxol (a product from Sigma-Aldrich) administered at 250 mg/kg |

Specifically, the test was conducted as follows. The test material was orally administered to the mice in the control group and the test groups, i.e., an aqueous solution of 2% gum arabic was administered to the mice in the negative control group, and 2,3,5-trimethylhydroquinone-1-hexyl ether (HTHQ), the test substance, was orally administered to the mice in the HTHQ-administered groups at 100 mg/kg body weight, 200 mg/kg body weight and 400 mg/kg body weight, respectively. Ambroxol (a product from Sigma-Aldrich) was orally administered to the mice in the ambroxol administered group at 250 mg/kg body weight. Here, ambroxol is a substance having expectoration action and has been selected as a positive control substance. 30 minutes after the oral administration of the test material, saline in which phenol red (a product from Sigma-Aldrich) was dissolved at a concentration of 0.05 g/mL was intraperitoneally administered at 15 mL/kg. Then, 30 minutes after the administration of phenol red, the mice were euthanized by inhalation of carbon dioxide, and the tracheae were extirpated. Given sites of the extirpated tracheae were cut into sections of fixed size to obtain trachea sections. The obtained trachea sections were added to centrifugation tubes, and 1 mL of saline was added there, and the mixture was sonicated using a ultrasonic washing machine for 15 minutes. After the centrifugation of 5 minutes at 10000 rpm, 0.5 mL of a upper layer solution was dispensed to the centrifugation tubes and 0.05 mL of 1 N sodium hydroxide was added to the tubes. After stirring with a vortex mixer, 0.2 mL of a sample was dispensed to a 96 well plate, and absorbance was measured at 546 nm using a micro plate reader (a product from BioTek Instruments, Inc). The amounts of phenol red excreted from the trachea sections of the mice in the control group and test groups were calculated by extrapolating the measured absorbance to the standard curve based on the absorbance of phenol red reference standard (75.0, 37.5, 18.8, 9.4, 7.4, 2.3 and 1.2 ng/mL). The amounts of phenol red excreted from the trachea sections were substituted in the following formula to calculate the sputum excretion ability of the test material. The formula to calculate the sputum excretion ability is as follows: "sputum excretion ability (%) = {(A/B) - 1} × 100" wherein A is the amounts of phenol red of the test material administered groups (an average) and B is the amount of phenol red of the negative control group (an average).

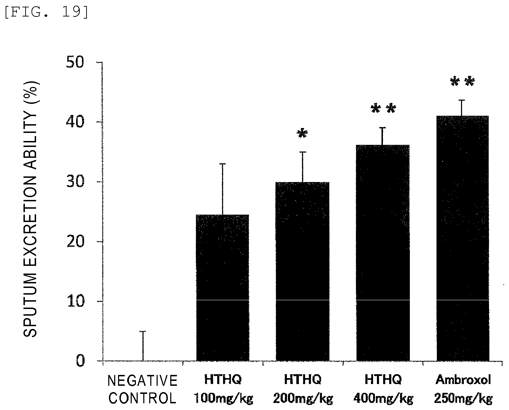

The result of this Example is shown in Figure 19. * described above bars in the graph of Figure 19 indicates that p value is < 0.05 in comparison to the negative control group, and ** indicates that p value is < 0.01 in comparison to the negative control group. The sputum excretion ability of the HTHQ-administered groups are 24.6% (HTHQ 100 mg/kg), 30.0% (HTHQ 200 mg/kg) and 36.2% (HTHQ 400 mg/kg), respectively, and significant increase compared to the negative control group was found in the 200 mg/kg administered group and 400 mg/kg administered group. Similarly, the sputum excretion ability of the ambroxol group, the positive control group, is 41.1% and thus significant increase compared to the negative control group was found. The above mentioned result proved that HTHQ has the action to promote sputum excretion same as ambroxol, i.e., expectoration action. Therefore, it was shown that HTHQ can promote the excretion of the sputum resulting from respiratory disease such as common cold and acute bronchitis as well as chronic respiratory disease such as COPD, asthma and interstitial pneumonia same as ambroxol and is effective in the treatment and improvement of these diseases.

### [Example 5]

### 5. Study of action on myocardial disorder induced by doxorubicin

Doxorubicin is used to produce disease-model animals of cardiomyopathy. The doxorubicin and 2,3,5-trimethylhydroquinone-1-hexyl ether (HTHQ) as the hydroquinone derivative represented by the general formula (1) described above of the present invention were simultaneously administered to female SD rats of 4 week old after birth to examine the effect of the action. The test groups consisted of the control group to which sterile saline was administered; test group 1 to which doxorubicin (13 mg/kg body weight) alone was administered; test group 2 to which doxorubicin (13 mg/kg body weight) and HTHQ (50 mg/kg body weight) were administered in combination; and test group 3 to which doxorubicin (13 mg/kg body weight), HTHQ (50 mg/kg body weight) and recombinant human erythropoietin (400 IU/kg body weight) were administered in combination. The recombinant human erythropoietin (rHuEPO) is mainly used in the treatment of renal anemia etc., and has the effect to protect myocardium. The test group 3 is used to confirm the presence or absence of the inhibitory effect brought about by combining HTHQ and rHuEPO on myocardial disorder.

Doxorubicin was orally administered in a single dose to the rats in test groups 1 to 3. 2,3,5-trimethylhydroquinone-1-hexyl ether (HTHQ) was orally administered to the rats in test groups 2 and 3 twice in total: 3 days before the administration of doxorubicin and on the day of the administration. Recombinant human erythropoietin was daily administered to the rats in test group 3 starting from 3 days before the administration of doxorubicin by intravenous injection. The number of animals in each group was 16. 8 animals of each group were sacrificed after collecting blood on day 7 and 14 after the administration of doxorubicin, and the autopsy of the hearts, the calculation of the relative weights of the hearts, the histopathologic examination of the hearts and the hematological examination were conducted.

### <Relative weights of the hearts>

Myocardium is hypertrophied by fibrosing and the weight of the myocardium increases, and the relative weight of the heart tends to increase. The result of the relative weights of the hearts of the rats in the control group and test groups 1 to 3 is shown in Table 5 below. The relative weight of the hearts of test group 2 was higher than that of test group 1 on day 7 after the administration of doxorubicin, but there was no significant difference between the relative weights of the hearts of test groups 2 and 1 on day 14 after the administration. Also, there was no significant difference between the other groups on day 7 and 14 after the administration (p < 0.05).

**[Table 5]**

| | | Day 7 after administration (%) | Day 14 after administration (%) |
|---|---|---|---|
| Control group | saline | 0.391±0.0506 | 0.3768±0.0231 |
| Test group 1 | doxorubicin | 0.3819±0.0204 | 0.3626±0.0221 |
| Test group 2 | doxorubicin + HTHQ | 0.4208±0.0409^{a} | 0.3658±0.0357 |
| Test group 3 | doxorubicin + HTHQ + rHuEPO | 0.3936±0.0347 | 0.3656±0.0256 |

| | | | |
|---|---|---|---|
| ^{a}; p<0.05 (In comparison to the value of Test group 1) | | | |

### <Autopsy of the hearts and gross pathology of the hearts>

The autopsy of the hearts was conducted and the gross pathology was observed. Apparent lesions were not observed in test groups 1 to 3, the doxorubicin administered groups, compared to the control group.

### <Findings from histopathologic examination 1 (Light microscope)>

The result of histopathologic examination of the hearts is shown in Table 6 below. "-" indicates "not found", "+" indicates "mild", "++" indicates "moderate", and "+++" indicates "severe" in severity scores of the table. Degeneration of myocardial cells, loss and disorganization of myocardial fibers, myocardial necrosis, loss of striations and cell infiltration in interstitial tissue etc. were observed in the cardiac tissue of test group 1 on day 7 after the administration of doxorubicin. However, these lesions were alleviated in the rats in test group 2 to which HTHQ was administered compared to the rats in test group 1, and these lesions were further alleviated in the rats in test group 3 to which HTHQ and rHuEPO were administered and the appearance of the myocardial tissue was close to normal myocardial tissue. The lesions of tissue on day 14 after the administration of doxorubicin have further progressed in test group 1 compared to those on day 7 after the administration, and advanced vacuole degeneration in myocardial cells and interstitial tissue, hypertrophy and atrophy of myocardial fibers, loss and disorganization of myocardial fibers, myocardial necrosis and loss of striations etc. were observed. The lesions of tissue of the rats in test groups 2 and 3 to which HTHQ was administered were also alleviated compared to those in test group 1 on day 14 after the administration, same as on day 7 after the administration.

**[Table 6]**

| Lesion | Severity score | | | | | |
|---|---|---|---|---|---|---|
| | Day 7 after administration | | | Day 14 after administration | | |
| | Test group 1 | Test group 2 | Test group 3 | Test group 1 | Test group 2 | Test group 3 |
| Degeneration of myocardial cells | ++ | + | + | +++ | + | + |
| Loss and disorganization of myocardial fibers | ++ | + | - | +++ | + | + |
| Loss of striations | ++ | + | + | +++ | + | + |
| Vacuolation of myocardial fibers | + | - | - | +++ | + | + |
| Loss of intercalated discs | + | + | - | ++ | + | - |
| Cell infiltration in interstitial tissue | + | - | - | - | - | - |

### <Findings from histopathologic examination 2 (Transmission electron microscope)>

Loss of striations, loss of intercalated discs, swelling of mitochondria, loss of mitochondrial outer membrane and detachment of cristae etc. were observed in the myocardial cells of test group 1 on day 7 after the administration of doxorubicin. However, loss of striations was alleviated and the myocardial cells and mitochondria were aligned almost uniformly in the rats in test group 2 and 3 to which HTHQ was administered, and thus the appearance of the myocardial cells of the rats in the groups was close to normal cells. The appearance similar to that on day 7 after the administration was observed in the myocardial cells of the rats in test groups 1 to 3 on day 14 after the administration of doxorubicin.

Findings from these histopathologic examinations showed that the hydroquinone derivative represented by the general formula (1) described above of the present invention has the effect to inhibit the cardiac fibrosis induced by doxorubicin and the effect to prevent or treat cardiomyopathy. It was proved that using this hydroquinone derivative and recombinant human erythropoietin in combination further improves the effects to inhibit or treat cardiac fibrosis.

### <Creatine phosphokinase ; CPK>

Creatine phosphokinase (CPK) is an enzyme which is distributed in muscle, brain and nerves in large numbers and is involved in energy metabolism. In particular, it is a clinically important index as an escape enzyme which effluxes into blood when skeletal muscle or myocardium is damaged. The creatine phosphokinase (CPK) value on day 14 after the administration of doxorubicin was measured and the result showed that the value of test groups 1 and 2 was significantly higher than that of the control group (p < 0.05) . However, the CPK value of the rats in test groups 2 and 3 to which HTHQ was administered was significantly lower compared to that of test group 1 (p < 0.05), and increase of CPK value was not seen in test group 3 to which HTHQ and rHuEPO were administered. The above mentioned result showed that the hydroquinone derivative represented by the general formula (1) has the action to inhibit the myocardial damage induced by doxorubicin and has the effect to prevent or treat cardiomyopathy. It was proved that using this hydroquinone derivative and recombinant human erythropoietin in combination further improves the effects to inhibit or treat myocardial disorder.

The present invention is not limited by the embodiments or Examples, and forms variously changed in design without departing from the contents of the present invention defined in the claims are included in the technical scope.

### Industrial Applicability

The present invention can inhibit or improve chronic respiratory disease such as chronic obstructive pulmonary disease (COPD), asthma and interstitial pneumonia, cardiomyopathy, and pulmonary or cardiac fibrosis induced by the administration of an agent such as an anticancer agent etc., and is useful in the prevention, treatment or improvement of a chronic respiratory disease, cardiomyopathy and pulmonary or cardiac fibrosis diseases caused by side effects of an agent such as an anticancer agent.

## Claims

1. A therapeutic agent for a chronic respiratory disease, comprising, as an active ingredient, a hydroquinone derivative represented by general formula (1) 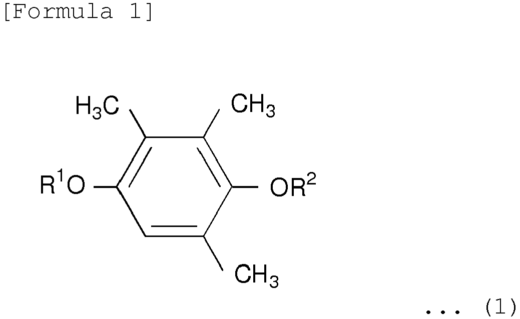 wherein R¹ represents an alkyl group having 4 to 8 carbon atoms, and R² represents a hydrogen atom, an alkylcarbonyl group having 2 to 6 carbon atoms or an alkoxycarbonyl group having 2 to 6 carbon atoms.

2. The therapeutic agent for a chronic respiratory disease according to claim 1, wherein the hydroquinone derivative is 2,3,5-trimethylhydroquinone-1-hexyl ether or 2,3,5-trimethylhydroquinone-1-hexyl ether 4-acetate.

3. The therapeutic agent for a chronic respiratory disease according to claim 1 or 2, wherein the chronic respiratory disease is at least one disease selected from the group consisting of chronic obstructive pulmonary disease (COPD), interstitial pneumonia and asthma.

4. The therapeutic agent for a chronic respiratory disease according to claim 1 or 2, wherein the chronic respiratory disease is chronic obstructive pulmonary disease (COPD).

5. The therapeutic agent for a chronic respiratory disease according to claim 3, wherein the interstitial pneumonia is caused by an agent.

6. The therapeutic agent for a chronic respiratory disease according to claim 5, wherein the agent is at least one agent selected from the group consisting of bleomycin, gefitinib, erlotinib, cetuximab, panitumumab, bortezomib, cisplatin, oxaliplatin, cyclophosphamide, azathioprine, tacrolimus, penicillamine, methotrexate, salazosulfapyridine, leflunomide, hydralazine, shosaikoto, amiodarone and interferon.

7. A food composition for prevention or improvement of a chronic respiratory disease, comprising, as an active ingredient, a hydroquinone derivative represented by general formula (1) 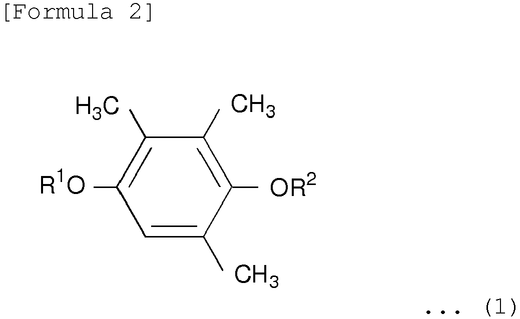 wherein R¹ represents an alkyl group having 4 to 8 carbon atoms, and R² represents a hydrogen atom, an alkylcarbonyl group having 2 to 6 carbon atoms or an alkoxycarbonyl group having 2 to 6 carbon atoms.

8. The food composition for prevention or improvement of a chronic respiratory disease according to claim 7, wherein the hydroquinone derivative is 2,3,5-trimethylhydroquinone-1-hexyl ether or 2,3,5-trimethylhydroquinone-1-hexyl ether 4-acetate.

9. The food composition for prevention or improvement of a chronic respiratory disease according to claim 7 or 8, wherein the chronic respiratory disease is at least one disease selected from the group consisting of chronic obstructive pulmonary disease (COPD), interstitial pneumonia and asthma.

10. The food composition for prevention or improvement of a chronic respiratory disease according to claim 7 or 8, wherein the chronic respiratory disease is chronic obstructive pulmonary disease (COPD).

11. A composition for inhibiting cardiac fibrosis, comprising, as an active ingredient, a hydroquinone derivative represented by general formula (1) 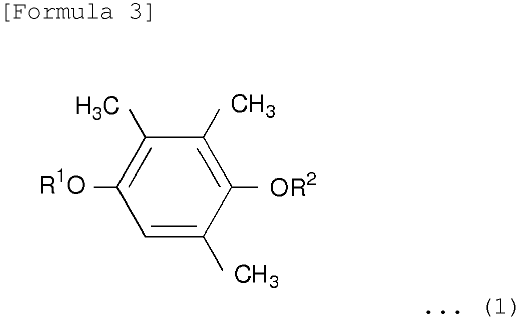 wherein R¹ represents an alkyl group having 4 to 8 carbon atoms, and R² represents a hydrogen atom, an alkylcarbonyl group having 2 to 6 carbon atoms or an alkoxycarbonyl group having 2 to 6 carbon atoms.

12. The composition for inhibiting cardiac fibrosis according to claim 11, wherein the hydroquinone derivative is 2,3,5-trimethylhydroquinone-1-hexyl ether or 2,3,5-trimethylhydroquinone-1-hexyl ether 4-acetate.

13. The composition for inhibiting cardiac fibrosis according to claim 11 or 12, wherein the cardiac fibrosis is caused by an agent.

14. The composition for inhibiting cardiac fibrosis according to claim 13, wherein the agent is an anthracyclin anticancer agent.
